# EUROPÄISCHE PATENTSCHRIFT

(11) **EP 0 500 108 B1**
(45) Veröffentlichungstag und Bekanntmachung des Hinweises auf die Patenterteilung: **16.10.1996**
(21) Anmeldenummer: 92102864.3
(22) Anmeldetag: 20.02.1992
(51) Int. Cl.: C12N 15/62, C12N 15/27, C07K 1/00, C12P 21/02, C07K 14/535

(54) **Verbesserte Aktivierung von rekombinanten Proteinen**
Improved activation of recombinant proteins
Activation améliorée de protéines recombinantes

(30) Priorität: 21.02.1991 DE 4105480
(43) Veröffentlichungstag der Anmeldung: 26.08.1992
(73) Patentinhaber: BOEHRINGER MANNHEIM GMBH, 68298 Mannheim (DE)
(72) Erfinder: Ambrosius, Dorothea, Dr. rer. nat., W-8127 Iffeldorf (DE); Dony, Carola, Dr. rer. nat., W-8130 Starnberg (DE); Rudolph, Rainer, Dr. rer. nat., W-8120 Weilheim (DE)
(74) Vertreter: Huber, Bernhard, Dipl.-Chem.

(56) Entgegenhaltungen:
- EP-A- 0 229 998
- BIOCHEMICAL AND BIOPHYSICAL RESEARCH COMMUNICATIONS, Bd. 159, Nr. 1, 28. Februar 1989, DULUTH, MINNESOTA US, Seiten 103-111; T. KUGA ET AL.: 'Mutagenesis of human granulocyte colony stimulating factor'
- ANGEWANDTE CHEMIE. INTERNATIONAL EDITION, Bd. 97, Nr. 8, 1985, WEINHEIM DE, Seiten 639-654; M. MUTTER: 'Die Konstruktion von neuen Proteinen und Enzymen - eine Zukunftsperspektive?'
- TIBTECH, Bd. 6, Mai 1988, CAMBRIDGE, UK, Seiten 95-101; J.F. KANE ET D.L. HARTLEY: 'Formation of recombinant protein inclusion bodies in Escherichia coli'

## Beschreibung

Die vorliegende Erfindung betrifft ein Verfahren zur Aktivierung von rekombinanten Proteinen, insbesondere zur Aktivierung von rekombinanten Proteinen aus Prokaryonten.

Bei der Expression von rekombinanten Proteinen in Prokaryonten entstehen die Proteine in der Wirtszelle oft als zumindest teilweise inaktive, schwerlösliche Aggregate (refractile bodies, inclusion bodies IB), die außerdem noch mit Proteinen der Wirtszelle verunreinigt sein können. Bevor derartige Proteine z.B. für therapeutische oder diagnostische Zwecke verwendet werden können, müssen sie in ihre aktive Form überführt werden.

Verfahren zur Renaturierung von rekombinanten Proteinen sind allgemein bekannt und beispielsweise in der EP-A 0 114 506, WO 86/00610, WO 84/03711, US 4,530,787 und der EP-A 0 241 022 offenbart. Mit diesen bekannten Verfahren werden jedoch häufig bei der Aktivierung von natürlichen Proteinsequenzen niedrige Ausbeuten erhalten. Das der vorliegenden Erfindung zugrundeliegende Problem besteht somit darin, eine Verbesserung bei den Renaturierungsausbeuten von rekombinanten Proteinen zu erzielen. Eine prinzielle Möglichkeit hierfür wäre die Bereitstellung eines Verfahrens, bei dem die Ausbeuten durch Wahl der Renaturierungsbedingungen verbessert werden könnten. Dies ist jedoch nicht Gegenstand der vorliegenden Erfindung.

Die Lösung der erfindungsgemäßen Aufgabe beruht auf der überraschenden Beobachtung, daß sich die Renaturierungsausbeute eines Proteins erhöht, wenn man an seinen N- oder/und C-Terminus zusätzliche Helfersequenzen anfügt.

Ein Gegenstand der vorliegenden Erfindung ist daher ein Verfahren zur Aktivierung von rekombinanten Proteinen, insbesondere von rekombinanten Proteinen aus Prokaryonten, die in einer zumindest teilweise inaktiven Form vorliegen, wobei dieses Verfahren dadurch gekennzeichnet ist, daß man durch an sich bekannte Solubilisierungs- oder/und Renaturierungstechniken ein Protein aktiviert, welches an seinem N- oder/und C-Terminus zusätzliche Helfersequenzen mit einer Länge von 2 bis 50 Aminosäuren enthält, wobei die relativen Hydrophobizitäten dieser Helfersequenzen, berechnet als Summe der in Tabelle 1 angegebenen relativen Hydrophobizitäten der einzelnen Aminosäuren, einen negativen Zahlenwert aufweisen.

Der Begriff "relative Hydrophobizität" im Sinne der vorliegenden Erfindung ist aus den Literaturstellen T.E. Creighton (1983), Proteins, Structure and Molecular Principles, W.H. Freeman and Company, New York, S. 142, Tabelle 4.4; G.von Heijne und C. Blomberg (1979) Eur.J.Biochem. 95, 175-181 und Y. Nozaki und C. Tanford (1971), J.Biol.Chem. 246, 2211-2217 abgeleitet. Die Bestimmung der Werte für die relative Hydrophobizität einer Aminosäure erfolgte dabei z.B. nach Nozaki/Tanford durch Ermittlung des Verteilungsgleichgewichts dieser Aminosäure zwischen einem nicht polaren Lösungsmittel (z.B. Ethanol/Dioxan) und Wasser. Die relative Hydrophobizität ist eine Energiegröße und somit in kcal/mol angegeben. Ein positiver Wert für die relative Hydrophobizität bedeutet, daß eine Präferenz für nicht polare Lösungsmittel vorliegt, d.h. daß es sich um eine unpolare Aminosäure handelt. Besitzt die relative Hydrophobizität hingegen einen Zahlenwert, der kleiner als 0 ist, so handelt es sich um eine polare Aminosäure, die eine Präferenz für Wasser gegenüber einem nicht polaren Lösungsmittel zeigt. Bei derartigen Aminosäuren wird folglich beim Übergang z.B. von Ethanol zu Wasser Energie frei.

Die folgende Tabelle 1 enthält eine Auflistung der Werte für die relative Hydrophobizität der einzelnen Aminosäuren.

**Tabelle 1**

| Aminosäuren | Relative Hydrophobizität (kcal/mol) |
|---|---|
| Gly | 0 |
| Leu | 1,8 |
| Ile | 2,5 |
| Val | 1,5 |
| Ala | 0,5 |
| Phe | 2,5 |
| Cys | - 2,8 |
| Met | 1,3 |
| Thr | 0,4 |
| Ser | - 0,3 |
| Trp | 3,4 |
| Tyr | 2,3 |
| Gln | - 0,3 |
| Lys | - 4,2 |
| Asn | - 0,2 |
| Glu | - 9,9 |
| His | 0,5 |
| Asp | - 7,4 |
| Arg | - 11,2 |
| Pro | - 3,3 |

Aus dieser Tabelle ist ersichtlich, daß die Aminosäuren Cystein, Prolin und insbesondere Glutamat, Aspartat, Arginin und Lysin eine stark negative relative Hydrophobizität aufweisen.

Überraschenderweise wurde festgestellt, daß durch Anfügung von Helfersequenzen mit einer Länge von 2 bis 50 Aminosäuren an eine Proteinsequenz die Aktivierung von rekombinanten Proteinen erheblich verbessert wird, wenn diese Helfersequenzen in ihrer Summe eine negative relative Hydrophobizität aufweisen. Vorzugsweise beträgt die Länge dieser Helfersequenzen 2 bis 20, besonders bevorzugt 5 bis 20 Aminosäuren.

Weiterhin bevorzugt ist, wenn für diese Helfersequenzen der Quotient aus relativer Hydrophobizität und Anzahl der Aminosäuren - 2,0 kcal/mol oder weniger, besonders bevorzugt von - 2,5 kcal/mol oder weniger und am meisten bevorzugt von - 2,8 kcal/mol oder weniger beträgt.

Die Anfügung der Helfersequenzen an das rekombinante Protein kann mittels üblicher Techniken auf dem Gebiet der Molekularbiologie erfolgen. Dies geschieht vorzugsweise dadurch, daß man an ein oder an beide Enden der für ein zu exprimierendes rekombinantes Protein kodierenden DNA-Sequenz eine Oligonukleotidsequenz anfügt, welche für eine oben beschriebene Protein-Helfersequenz mit einer negativen relativen Hydrophobizität kodiert. Zu diesem Zweck werden beispielsweise DNA-Fragmente aus dem zu exprimierenden Gen isoliert, die einen Bereich enthalten, welcher für den Anfang bzw. das Ende des entsprechenden Gens kodiert. In diese DNA-Fragmente können dann z.B. unter Verwendung anderer Restriktionsschnittstellen synthetische Oligonukleotide eingefügt werden, welche für die Helfersequenzen kodierende Bereiche enthalten. Eine andere Möglichkeit besteht darin, die natürlichen DNA-Fragmente aus dem Gen vollständig durch Oligonukleotidsequenzen zu ersetzen. Auf diese Weise können modifizierte DNA-Sequenzen erhalten werden, welche zusätzlich zur Information eines rekombinanten Proteins die Information für die angefügten Helfersequenzen enthalten.

Als DNA-Sequenzen, welche für die N-terminalen Helfersequenzen kodieren, werden zweckmäßig DNA-Sequenzen verwendet, deren codon usage dem Expressionsorganismus angepaßt ist (E.L. Winnacker, Gene und Klone, Verlag Chemie, 1985, 224-241).

Für E.coli als Expressionsorganismus werden dabei für die folgenden Aminosäuren bevorzugt als Codons verwendet:

| | |
|---|---|
| Threonin | ACA |
| Prolin | CCA |
| Leucin | CTA |
| Lysin | AAA |
| Alanin | GCC |
| Glutaminsäure | GAA. |

Eine auf solche Weise modifizierte DNA, welche für ein rekombinantes Protein mit angefügten Helfersequenzen kodiert, wird durch Transformation in eine Wirtszelle, vorzugsweise in eine prokaryontische Zelle, besonders bevorzugt in eine E.coli-Zelle eingeführt. Anschließend erfolgt eine Kultivierung der transformierten Zellen in einem geeigneten Medium, Aufschluß der Zellen und Isolierung des in einem zumindest teilweise inaktiven Zustand entstandenen rekombinanten Proteins, das insbesondere in Form von Inclusion bodies vorliegt. Anschließend erfolgt eine Solubilisierung und Renaturierung dieses Proteins, günstigerweise bei einem pH-Wert, bei dem das Protein seine native Konformation einnehmen kann. Diese Verfahrensschritte können entsprechend bereits bekannter Techniken durchgeführt werden, wie sie etwa z.B. aus dem in der Beschreibungseinleitung genannten Stand der Technik zu entnehmen sind. Vorzugsweise erfolgt die Aktivierung des Proteins mittels einer Pulsrenaturierung, wie sie z.B. aus der EP-A 0 241 022 bekannt ist. Die durch die vorliegende Erfindung erzielte Verbesserung der bekannten Verfahrenstechniken beruht insbesondere auf der Anwesenheit der Helfersequenzen, die an den N- oder/und C-Terminus des rekombinanten Proteins angefügt sind. Dabei sind die Helfersequenzen vorzugsweise an den N-Terminus des zu aktivierenden Proteins angefügt. Die Anfügung von Helfersequenzen an den C-Terminus bringt jedoch auch positive Ergebnisse.

Als Helfersequenzen sind solche bevorzugt, die mindestens 2 Aminosäuren aus der Gruppe, bestehend aus Glutamat, Aspartat, Lysin, Arginin und Prolin enthalten, wobei Lysin-und Glutamatreste besonders bevorzugt und Glutamatreste am meisten bevorzugt sind. Weiterhin besonders bevorzugt ist, wenn die Helfersequenzen zwei direkt aufeinanderfolgende der oben genannten geladenen Aminosäuren (d.h. Glutamat, Aspartat, Lysin und Arginin) mit gleichnamiger Ladung, vorzugsweise zwei direkt aufeinanderfolgende Lysin- oder Glutamatreste, besonders bevorzugt zwei direkt aufeinanderfolgende Glutamatreste enthalten.

Bei späterer therapeutischer Verwendung der durch das erfindungsgemäße Verfahren erzeugten rekombinanten Proteine ist es vorteilhaft, daß sie eine Spaltstelle am Übergang zwischen den Helfersequenzen und dem erwünschten Protein aufweisen. Dadurch kann das Protein in seiner natürlichen Aminosäuresequenz erhalten werden. Diese Spaltstelle kann eine Sequenz sein, die von einer Protease oder einem chemischen Proteinspaltungsreagenz (z.B. BrCN) erkannt wird, wobei eine Proteasespaltstelle bevorzugt ist. Besonders bevorzugt handelt es sich bei der Spaltstelle um eine IgA-Proteasespaltstelle, wie sie in der WO 91/11520 beschrieben ist. Die genauen Spaltungsbedingungen sind ebenfalls der WO 91/11520 zu entnehmen. Weiterhin bevorzugt ist auch eine Spaltstelle, die eine Spaltstelle für den Faktor Xa ist.

Eine derartige Spaltstelle in der Proteinsequenz ist bei einer Verwendung des rekombinanten Proteins in der Analytik nicht erforderlich.

Konkrete Beispiele für Helfersequenzen, die zur Verbesserung der Protein-Aktivierung geeignet sind, sind die im folgenden an den N-Terminus eines Proteins angefügten Sequenzen:

Met-Glu (SEQ ID NO: 1)
Met-Thr-Pro-Leu-Pro-Arg-Pro-Pro (SEQ ID NO: 2)
Met-Thr-Pro-Leu-His-His-Pro-Arg-Pro-Pro (SEQ ID NO: 3)
Met-Thr-Pro-Leu-Lys-Lys-Pro-Arg-Pro-Pro (SEQ ID NO: 4)
Met-Thr-Pro-Leu-Glu-Glu-Gly-Pro-Arg-Pro-Pro
(SEQ ID NO: 5)
Met-Thr-Pro-Leu-Glu-Glu-Gly-Thr-Pro-Leu-Pro-Arg-Pro-Pro
(SEQ ID NO: 6)
Met-Thr-Pro-Leu-Glu-Glu-Gln-Pro-Pro (SEQ ID NO: 7)
Met-Lys-Ala-Lys-Arg-Phe-Lys-Lys-His-Pro-Arg-Pro-Pro
(SEQ ID NO: 8)
Met-Thr-Pro-Leu-Glu-Glu-Gly-Ile-Glu-Gly-Arg
(SEQ ID NO: 9)
Met Thr-Pro-Leu-Lys-Ala-Lys-Arg-Phe-Lys-Lys-His-Pro-Arg-
Pro-Pro (SEQ ID NO: 10)

Die Helfersequenzen SEQ ID NO: 5, 6, 7 und 9 mit 2 aufeinanderfolgenden Glutamatresten ergeben die höchsten Renaturierungsausbeuten und sind daher am meisten bevorzugt.

Das erfindungsgemäße Verfahren ist insbesondere zur Aktivierung von in Prokaryonten hergestellten rekombinanten Human-Proteinen und deren Derivaten, wie z.B. Plasminogen-Aktivatoren, Interferone, Interleukine und Granulozyten-Kolonien-stimulierende Faktoren geeignet. Besonders bevorzugt handelt es sich bei dem zu aktivierenden Protein um einen Granulozyten-Kolonien-stimulierenden Faktor (G-CSF), welcher die Anfangs-DNA-Sequenz ACACCA aufweist. Ebenso bevorzugt sind auch Derivate von G-CSF, die in der EP-A 0 456 200 offenbart sind.

Der Vektor pKK177-3-G-CSF Bg wurde am 28.03.1990 bei der Deutschen Sammlung für Mikroorganismen, Grisebachstr. 8, D-3400 Göttingen unter der Nummer DSM 5867 hinterlegt.

Die Erfindung soll weiterhin durch die folgenden Beispiele und Abbildungen verdeutlicht werden.

Figur 1 zeigt die Konzentrationsabhängigkeit der Renaturierungsausbeute (Argininkonzentration 0,2 mol/l) für Konstrukte, welche die Sequenzen analog Sequenz 0 von Tabelle 2 (Kurve 1), SEQ ID NO: 3 (Kurve 2), SEQ ID NO: 5 (Kurve 3) und SEQ ID NO: 8 (Kurve 4) enthalten.

Figur 2 zeigt die Konzentrationsabhängigkeit der Renaturierungsausbeute (Argininkonzentration 0,8 mol/l) für Konstrukte, welche die Sequenzen analog Sequenz 0 von Tabelle 2 (Kurve 1), SEQ ID NO: 3 (Kurve 2), SEQ ID NO: 5 (Kurve 3) und SEQ ID NO: 8 (Kurve 4) enthalten.

Figur 3 zeigt die Abhängigkeit der Renaturierungsausbeute von der Argininkonzentration (Kurvenbezeichnungen analog Figur 1 und 2).

Figur 4 zeigt die Reaktivierungsausbeute in Abhängigkeit von der Inkubationszeit (Argininkonzentration 0,2 mol/l, Kurvenbezeichnungen analog Figur 1 und 2).

### Beispiel 1

### Konstruktion der Vektoren

Der Vektor pKK177-3 G-CSF Bg (DSM 5867) wird mit EcoRI (partial) und ApaI verdaut und das Oligonukleotid 
EcoRI                                                    ApaI
    AATTCGGAGGAAAAATTA|ATG .........|ACACCACTGGGCC
                      |             |
                      |Met..........|G-CSF-Sequenz ohne ATG
 in das entstandene linearisierte Vektorfragment (ca. 3450 bp) insertiert.

AATTCGGAGGAAAAATTA: SEQ ID NO: 11
ACACCACTGGGCC: SEQ ID NO: 12

Die in die Lücke jeweils eingesetzte DNA-Sequenz entspricht dem genetischen Code für die in Tabelle 2 genannten Aminosäuren, d.h. beispielsweise für Konstrukt (2), wurde ein Oligonukleotid mit dem genetischen Code für Met-Thr-Pro-Leu-Pro-Arg-Pro-Pro (SEQ ID NO: 2) eingesetzt. Die durch Ligation der Oligonukleotide in den geschnittenen Vektor resultierenden Plasmide werden in E.coli HB101 transformiert. Um eine bessere Regulierbarkeit des tac-Promotors zu gewährleisten, wurden die Zellen zusätzlich mit einem zu pBP010 (Herstellung vgl. Europäische Patentanmeldung Nr. 91 111 155.7) kompatiblen Plasmid transformiert, das das lacI^{q}-Gen enthält. Das lacI^{q}-Gen ist dem Fachmann schon lange bekannt und leicht erhältlich. Als kompatible Plasmide zu pBP010 sind z.B. pACYC 177 (DSM 3693P), in welches das lacI^{q}-Gen insertiert ist, oder davon abgeleitete Plasmide geeignet (vgl. z.B. Gene 85 (1989), 109-114 und EP-A 0 373 365). Die resultierenden Klone werden auf Kanamycin (50 µg/ml)/Ampicillin (50 µg/ml) selektioniert und über Restriktionsanalyse identifiziert. Beim Schnitt mit EcoRI und EcoRV ergeben sich Fragmente der Längen ca. 3,15 kb, ca. 0,3 kb (mit den jeweiligen Konstrukten) und 4,85 kb.

### Beispiel 2

a) Fermentation:
   Die gemäß Beispiel 1 positiv identifizierten Klone werden in 5 ml-Kultur in LB-Medium mit Kanamycin und Ampicillin (Konzentrationen s. Beispiel 1) bis zu einer OD₅₅₀ von 0,5 angezogen und mit 5 mmol/l IPTG induziert und 3 Stunden bei 37°C inkubiert. 10 OD von dieser induzierten Kultur werden geerntet und daraus ein Gesamtzellextrakt hergestellt. Der Gesamtzellextrakt wird auf einem SDS-Page Gel analysiert.
   Wenn daraus ersichtlich ist, daß das gewünschte Protein exprimiert wird, wird die Kultur im 1 Liter-Maßstab wiederholt, die Zellen geerntet und eine IB-Präparation durchgeführt.
b) IB-Präparation:
   Die Zellen werden durch Zentrifugation geerntet, in 100 ml Tris-Magnesiumpuffer (10 mmol/l Tris, pH 8,0, 1 mmol/l MgCl₂) aufgenommen und mit Lysozym (0,3 mg/ml) aufgeschlossen.
   Es wird 15 Minuten bei 37°C inkubiert und ein French-Press-Durchgang durchgeführt (1200 psi). Anschließend erfolgt ein DNAse-Verdau (2 mg DNAse I) bei 30 Minuten und 37°C.
   Es werden 20 ml 0,5 mol/l NaCl, 20 mmol/l EDTA, pH 8,0 und 3 ml 20 % Triton X 100 zugegeben und 10 Minuten bei Raumtemperatur inkubiert.
   Die Suspension wird 10 Minuten bei 15.000 Upm und 4°C zentrifugiert. Das Pellet wird in 30 ml 50 mmol/l Tris, pH 8,0, 50 mmol/l EDTA und 0,5 % Triton X 100 aufgenommen und mit Ultraschall behandelt. Es wird wieder zentrifugiert, resuspendiert und mit Ultraschall behandelt. Diese Prozedur wird noch zweimal wiederholt. Anschließend wird zentrifugiert und die so erhaltenen Pellets als IBs im Beispiel 3 eingesetzt.

### Beispiel 3

### Solubilisierung/Renaturierung

a) Solubilisierung
   Solubilisierungspuffer:
   6 mol/l Guanidin-Hydrochlorid
   0,1 mol/l Tris-Puffer, pH 8,0
   1 mmol/l EDTA
   100 mmol/l DTE (Dithioerythritiol)

   Dialysepuffer 1:
   6 mol/l Guanidin-Hydrochlorid
   3 mmol/l EDTA bei pH 3,0

   1 g Inclusion Bodies werden zu 30 ml Solubilisierngspuffer gegeben, 5 Minuten mit Ultraschall homogenisiert und eine Stunde bei Raumtemperatur inkubiert. Es wird HCl zugegeben, bis der pH-Wert 3,0 erreicht ist. Anschließend wird unlösliches Material abzentrifugiert.
   Es wird gegen Dialysepuffer 1 dialysiert, bis DTE vollständig (≤ 1 mmol/l DTE) entfernt ist.
b) Pulsreaktivierung:
   Renaturierungspuffer:
   0,8 mol/l Arginin-Hydrochlorid
   0,1 mol/l Tris-Puffer, pH 8,0
   0,5 mmol/l GSH
   0,5 mmol/l GSSG
   1 mmol/l EDTA

   Dialysepuffer 2:
   10 mmol/l Tris-Puffer, pH 8,0
   1 mmol/l EDTA

Die Pulsreaktivierung erfolgt wie in EP-A 0 241 022 beschrieben. Es wird eine Vorrichtung gemäß Fig. 5 von EP-A 0 241 022 verwendet.

Dazu wird in Zeitintervallen von 30 Minuten Protein in das Reaktionsvolumen (100 ml Renaturierungspuffer) gegeben, so daß die Proteinkonzentration im Reaktionsvolumen pro Puls um 50 µg/ml steigt. Insgesamt wird 20 mal gepulst (Endkonzentration ca. 1 mg/ml Reaktionsvolumen).

Nach der Pulsreaktivierung werden aus dem Reaktionsvolumen Trübungen abzentrifugiert und das gesamte Reaktionsvolumen gegen Dialysepuffer 2, bis zur Entfernung von Arginin (≤ 50 mmol/l) dialysiert. (Die Überprüfung erfolgt zweckmäßig durch Leitfähigkeitsmessung. Die Dialyse kann beendet werden, wenn die Leitfähigkeit von Dialysepuffer und Reaktionsvolumen identisch ist.) Die Reaktivierungsausbeuten für die einzelnen Konstrukte, die mittels Aktivitätstest bestimmt wurden, zeigt Tabelle 2.

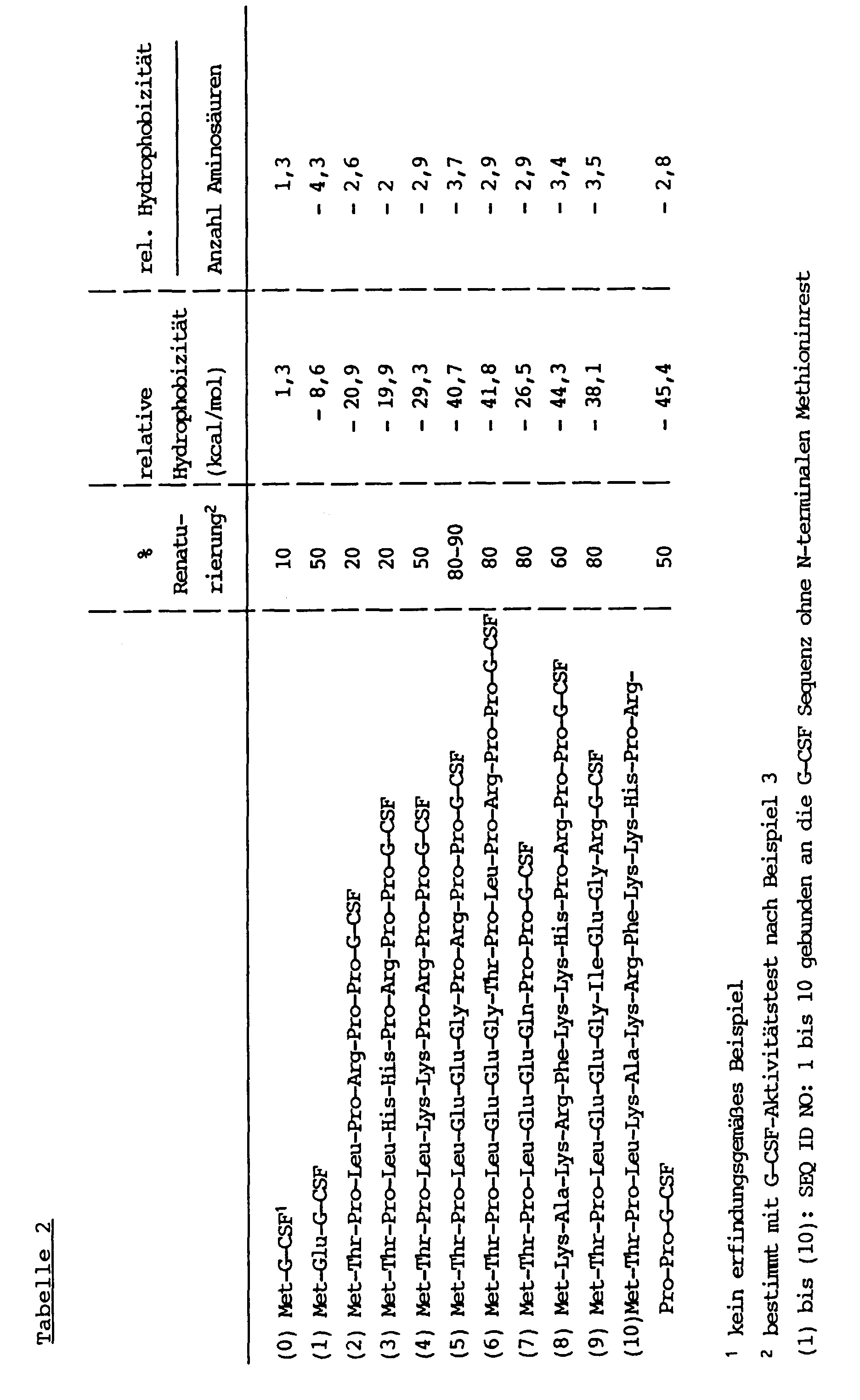

### Beispiel 4

### Bestimmung der Aktivität von G-CSF

Die Aktivität von G-CSF wird mit der murinen Leukämie-Linie NFS60, die vollkommen G-CSF-abhängig ist, wie in Biochem.J. 253 (1988) 213-218, Exp.Hematol. 17 (1989) 116-119, Proc. Natl.Acad.Sci. USA 83 (1986) 5010 beschrieben, getestet. Damit die Faktorabhängigkeit der Zellen erhalten bleibt, enthält das Medium (RPMI Medium, Boehringer Mannheim GmbH, Best.Nr. 2099445 mit 10 % fötalem Kälberserum) der Erhaltungskultur permanent 1000 U/ml G-CSF.

Gemessen wird bei diesem Test direkt die von G-CSF-stimulierte Proliferation der NFS60-Zellen durch den Einbau von ³H-Thymidin. Die Durchführung des Tests erfolgt folgendermaßen:

NFS60-Zellen, die sich in der exponentiellen Wachstumsphase befinden (Zelldichte maximal 1x10⁵ Zellen/ml) werden in Mikrotiterplatten überführt (1x10⁴ Zellen/Loch) und mit einer abnehmenden G-CSF-Konzentration kultiviert. Die maximale Dosis G-CSF in Loch 1 entspricht der Konzentration in der Erhaltungskultur (1000 U/ml, spezifische Aktivität 1x10⁸ U/mg Protein). Die Verdünnung erfolgt in Zehnerschritten.

Nach etwa 24 Stunden Inkubation erfolgt die Zugabe von ³H-Thymidin (0,1 µCi/Loch). Daraufhin werden die Zellen noch weitere 16 Stunden inkubiert.

Für die Auswertung der Tests werden die Zellen in der Mikrotiterplatte eingefroren, so daß sie lysiert werden. Das Zell-Lysat wird auf einen Glasfaserfilter gesaugt, gespült, getrocknet und im Szintillationszähler gemessen. Der Einbau von ³H-Thymidin ist proportional zur G-CSF induzierten Proliferation der NFS60-Zellen.

### Beispiel 5

Bestimmung der Renaturierungsausbeute in Abhängigkeit von der Konzentration an denaturiertem Protein bei einmaliger Zugabe.

Ausgangsmaterial: Inclusions bodies der Konstruktionen Nr. 0, 3, 5 und 8 der Tabelle 2.

### Solubilisierung und 1. Dialyse:

Das IB-Material wurde analog Beispiel 3 solubilisiert, zur Entfernung des Reduktionsmittels dialysiert und anschließend auf eine Proteinkonzentration von 30 mg/ml eingestellt (M.M. Bradford, Anal. Biochem. 72 (1976), 255).

### Renaturierung:

Die Reaktivierung erfolgte in 0,8 mol/l bzw. 0,2 mol/l Arginin-Hydrochlorid, 10 mmol/l EDTA, 0,5 mmol/l GSH und 0,5 mmol/l GSSG bei 20°C und pH 8,0.

In die jeweiligen Renaturierungsansätze wurden Proteinkonzentrationen zwischen 0,3 und 3 mg/ml eingestellt. Die Konzentration von Guanidin-Hydrochlorid betrug in allen Ansätzen 0,55 mol/l.
Nach einer Inkubation von 3 h bei Raumtemperatur wurde die Reaktion durch Ansäuern (pH 4,5) gestoppt.

Das Verhältnis von denaturiertem und renaturiertem Protein wurde durch HPLC ermittelt.
- Laufpuffer A:: 0,12 % Trifluoressigsäure (v/v)
- Laufpuffer B:: 90 % Acetonitril (v/v), 0,1 % Triflouressigsäure (v/v)
- Gradient von B:: 40 bis 70 % in 30 min
- Fluß:: 1 ml/min, Detektion bei 280 nm

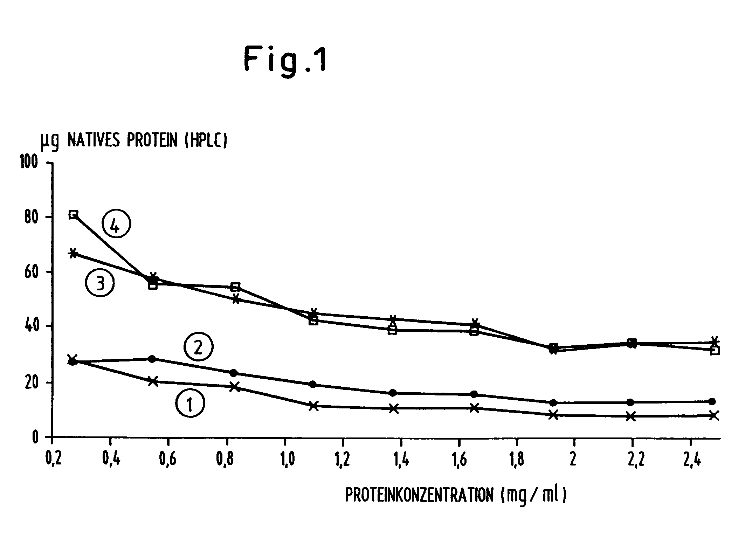

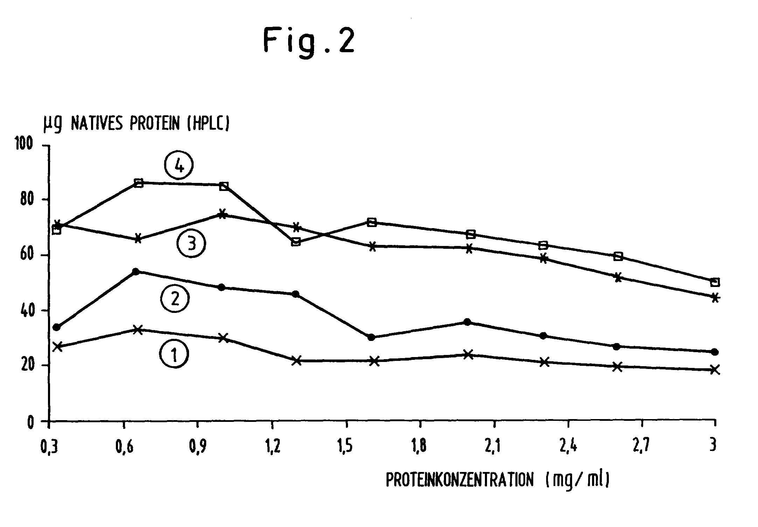

Die Ergebnisse sind in Figur 1 und 2 dargestellt.

### Beispiel 6

### Renaturierung in Abhängigkeit von der Arginin-Konzentration

Als Ausgangsmaterial dienten dialysierte Solubilisate (Proteinkonzentration von 10 mg/ml) der Konstruktionen 0, 3, 5 und 8 (Tab. 2), die analog Beispiel 5 hergestellt wurden.

Durch eine einmalige Zugabe an denaturiertem Protein wurde die Proteinkonzentration im Renaturierungspuffer (0 bis 0,8 mol/l Arginin-Hydrochlorid, 100 mmol/l Tris, 10 mmol/l EDTA, 0,5 mmol/l GSH, 0,5 mmol/l GSSG, Raumtemperatur bei pH 8) auf 1 mg/ml eingestellt.

Nach einer Inkubationszeit von 3 h wurde die Reaktion durch Ansäuern (pH 4,5) gestoppt. Die anschließende Auswertung erfolgte durch HPLC analog Beispiel 5.

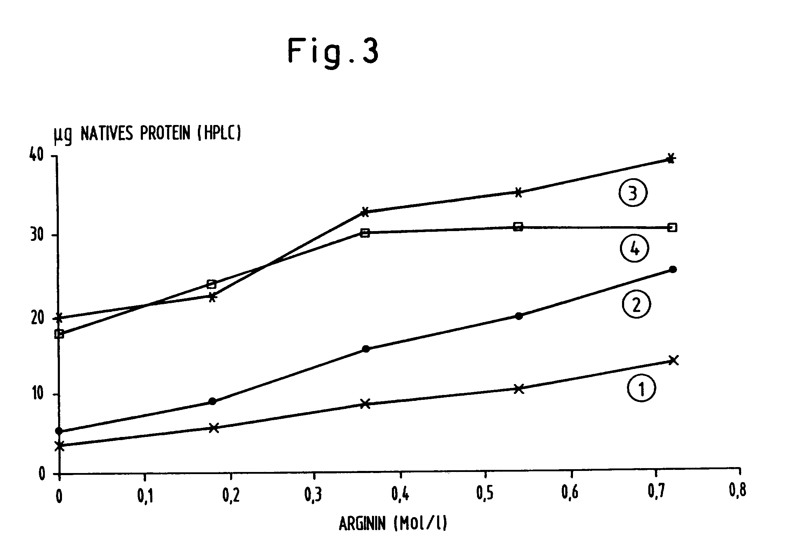

Figur 3 zeigt die Ergebnisse.

### Beispiel 7

### Kinetik der Reaktivierung in 0,2 mol/l Arginin-Puffer

Ausgangsmaterial: Es wurden die Solubilisate aus Beispiel 6 eingesetzt.

Die Reaktivierung erfolgte in 0,2 mol/l Arginin-Hydrochlorid, 100 mmol/l Tris, 10 mmol/l EDTA, 0,5 mmol/l GSH, 0,5 mmol/l GSSG, bei Raumtemperatur pH 8. Die Proteinkonzentration im Reaktionsansatz wurde durch eine einmalige Zugabe auf 1 mg/ml und die Guanidin-Konzentration auf 0,55 mol/l eingestellt. Nach 5, 10, 15, 60 und 180 Minuten wurden Proben entnommen, die Reaktion jeweils durch Ansäuern (pH 4,5) gestoppt und anschließend durch HPLC die Reaktivierungskenetik erfaßt (vgl. Beispiel 5).

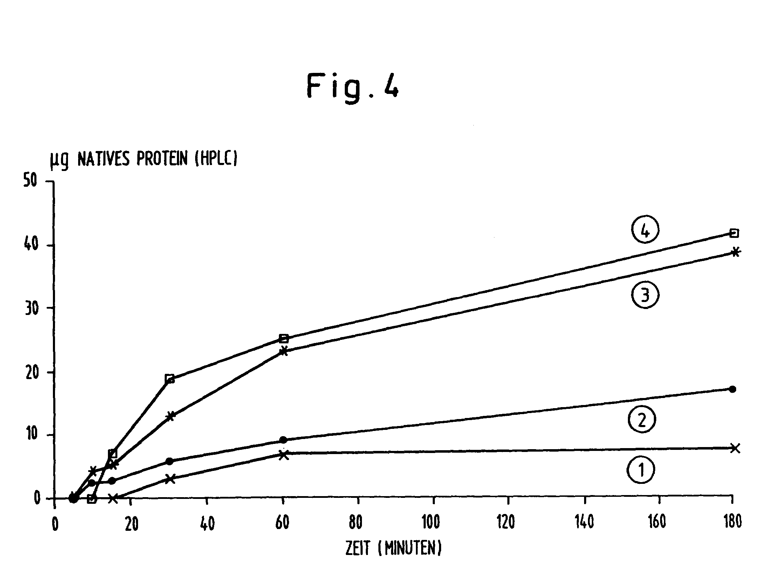

Figur 4 zeigt die Reaktivierungsausbeute in Abhängigkeit von der Inkubationszeit.

### SEQUENZLISTE

SEQ ID NO:1:
   - Länge:: 2 Aminosäuren
   - Typ:: Aminosäure
   - Topologie:: linear 
Met Glu
1
SEQ ID NO:2:
   - Länge:: 8 Aminosäuren
   - Typ:: Aminosäure
   - Topologie:: linear 
Met Thr Pro Leu Pro Arg Pro Pro
1               5
SEQ ID NO:3:
   - Länge:: 10 Aminosäuren
   - Typ:: Aminosäure
   - Topologie:: linear 
Met Thr Pro Leu His His Pro Arg Pro Pro
1               5                   10
SEQ ID NO:4:
   - Länge:: 10 Aminosäuren
   - Typ:: Aminosäure
   - Topologie:: linear 
Met Thr Pro Leu Lys Lys Pro Arg Pro Pro
1               5                   10
SEQ ID NO:5:
   - Länge:: 11 Aminosäuren
   - Typ:: Amiosäure
   - Topologie:: linear 
Met Thr Pro Leu Glu Glu Gly Pro Arg Pro Pro
1               5                   10
SEQ ID NO:6:
   - Länge:: 14 Aminosäuren
   - Typ:: Aminosäure
   - Topologie:: linear 
Met Thr Pro Leu Glu Glu Gly Thr Pro Leu Pro Arg Pro Pro
1               5                   10
SEQ ID NO:7:
   - Länge:: 9 Aminosäuren
   - Typ:: Aminosäure
   - Topologie:: linear 
Met Thr Pro Leu Glu Glu Gln Pro Pro
1               5
SEQ ID NO:8:
   - Länge:: 13 Aminosäuren
   - Typ:: Aminosäure
   - Topologie:: linear 
Met Lys Ala Lys Arg Phe Lys Lys His Pro Arg Pro Pro
1               5                   10
SEQ ID NO:9:
   - Länge:: 11 Aminosäuren
   - Typ:: Aminosäure
   - Topologie:: linear 
Met Thr Pro Leu Glu Glu Gly Ile Glu Gly Arg
1               5                   10
SEQ ID NO:10:
   - Länge:: 16 Aminosäuren
   - Typ:: Aminosäure
   - Topologie:: linear 
Met Thr Pro Leu Lys Ala Lys Arg Phe Lys Lys His Pro Arg Pro Pro
1               5                   10                  15
SEQ ID NO:11:
   - Länge:: 18 Basenpaare
   - Typ:: Nukleinsäure
   - Strangform:: einzel
   - Topologie:: linear AATTCGGAGG AAAAATTA 18
SEQ ID NO:12:
   - Länge:: 13 Basenpaare
   - Typ:: Nukleinsäure
   - Strangform:: einzel
   - Topologie:: linear

ACACCACTGG GCC 13

## Patentansprüche

1. Verfahren zur Aktivierung von rekombinanten Proteinen, die in einer zumindest teilweise inaktiven Form vorliegen,
**dadurch gekennzeichnet,**
daß man durch an sich bekannte Solubilisierungs-oder/und Renaturierungstechniken ein Protein aktiviert, welches an seinem N- oder/und C-Terminus zusätzliche Helfersequenzen mit einer Länge von 2 bis 50 Aminosäuren enthält, wobei die relativen Hydrophobizitäten dieser Helfersequenzen, berechnet als Summe der in Tabelle 1 angegebenen relativen Hydrophobizitäten der einzelnen Aminosäuren, einen negativen Zahlenwert aufweisen.

2. Verfahren nach Anspruch 1,
**dadurch gekennzeichnet,**
daß die Helfersequenzen einen Wert für das Verhältnis von relativer Hydrophobizität zur Anzahl der Aminosäuren von -2,0 kcal/mol oder weniger aufweisen.

3. Verfahren nach einem der vorhergehenden Ansprüche,
**dadurch gekennzeichnet,**
daß die Helfersequenzen an den N-Terminus des zu aktivierenden Proteins angefügt sind.

4. Verfahren nach Anspruche 1 oder 2,
**dadurch gekennzeichnet,**
daß die Helfersequenzen an den C-Terminus des zu aktivierenden Proteins angefügt sind.

5. Verfahren nach einem der vorhergehenden Ansprüche,
**dadurch gekennzeichnet,**
daß die Helfersequenzen mindestens 2 Aminosäuren aus der Gruppe, bestehend aus Glutamat, Aspartat, Lysin, Arginin und Prolin enthalten.

6. Verfahren nach Anspruch 5,
**dadurch gekennzeichnet,**
daß die Helfersequenzen mindestens 2 Glutamat-Reste enthalten.

7. Verfahren nach Anspruch 5 oder 6,
**dadurch gekennzeichnet,**
daß die Helfersequenzen zwei direkt aufeinanderfolgende gleichnamig geladene Aminosäuren aus der Gruppe, bestehend aus Glutamat, Aspartat, Lysin und Arginin enthalten.

8. Verfahren nach Anspruch 7,
**dadurch gekennzeichnet,**
daß die Helfersequenzen zwei direkt aufeinanderfolgende Glutamat-Reste enthalten.

9. Verfahren nach einem der vorhergehenden Ansprüche,
**dadurch gekennzeichnet,**
daß man Proteine aktiviert, die eine Spaltstelle am Übergang zwischen den Helfersequenzen und dem erwünschten Protein aufweisen.

10. Verfahren nach Anspruch 9,
**dadurch gekennzeichnet,**
daß die Spaltstelle eine IgA-Protease-Spaltstelle ist.

11. Verfahren nach einem der vorhergehenden Ansprüche,
**dadurch gekennzeichnet,**
daß man ein Protein aktiviert, das eine N-terminale Helfersequenz mit einer im folgenden angegebenen Sequenzen enthält:

Met-Glu (SEQ ID NO: 1)
Met-Thr-Pro-Leu-Pro-Arg-Pro-Pro (SEQ ID NO: 2)
Met-Thr-Pro-Leu-His-His-Pro-Arg-Pro-Pro (SEQ ID NO: 3)
Met-Thr-Pro-Leu-Lys-Lys-Pro-Arg-Pro-Pro (SEQ ID NO: 4)
Met-Thr-Pro-Leu-Glu-Glu-Gly-Pro-Arg-Pro-Pro
(SEQ ID NO: 5)
Met-Thr-Pro-Leu-Glu-Glu-Gly-Thr-Pro-Leu-Pro-Arg-Pro-
Pro (SEQ ID NO: 6)
Met-Thr-Pro-Leu-Glu-Glu-Gln-Pro-Pro (SEQ ID NO: 7)
Met-Lys-Ala-Lys-Arg-Phe-Lys-Lys-His-Pro-Arg-Pro-Pro
(SEQ ID NO: 8)
Met-Thr-Pro-Leu-Glu-Glu-Gly-Ile-Glu-Gly-Arg
(SEQ ID NO: 9)
Met-Thr-Pro-Leu-Lys-Ala-Lys-Arg-Phe-Lys-Lys-His-Pro-
Arg-Pro-Pro (SEQ ID NO: 10)

12. Verfahren nach einem der vorhergehenden Ansprüche,
**dadurch gekennzeichnet,**
daß man ein Protein aktiviert, bei dem es sich um Granulozyten-Kolonien stimulierenden Faktor (G-CSF) oder um Derivate davon handelt.

## Claims

1. Process for the activation of recombinant proteins which are present in at least a partially inactive form,
**wherein**
a protein is activated by known solubilization or/and renaturation techniques, said protein having additional helper sequences 2 to 50 amino acids in length at its N- or/and C-terminus whereby the relative hydrophobicity of these helper sequences, which is calculated as the sum of the relative hydrophobicities specified in Table 1 for the individual amino acids, has a negative numerical value.

2. Process as claimed in claim 1,
**wherein**
the helper sequences have a value for the ratio of relative hydrophobicity to the number of amino acids which is -2.0 kcal/mol or less.

3. Process as claimed in one of the previous claims,
**wherein**
the helper sequences are added to the N-terminus of the protein to be activated.

4. Process as claimed in one of the claims 1 to 2,
**wherein**
the helper sequences are added to the C-terminus of the protein to be activated.

5. Process as claimed in one of the previous claims,
**wherein**
the helper sequences contain at least two amino acids from the group comprising glutamate, aspartate, lysine, arginine and proline.

6. Process as claimed in claim 5,
**wherein**
the helper sequences contain at least two glutamate residues.

7. Process as claimed in claim 5 or 6,
**wherein**
the helper sequences contain two directly successive amino acids having a charge of the same kind from the group comprising glutamate, aspartate, lysine and arginine.

8. Process as claimed in claim 7,
**wherein**
the helper sequences contain two directly successive glutamate residues.

9. Process as claimed in one of the previous claims,
**wherein**
proteins are activated which have a cleavage site at the junction between the helper sequences and the desired protein.

10. Process as claimed in claim 9,
**wherein**
the cleavage site is an IgA protease cleavage site.

11. Process as claimed in one of the previous claims,
**wherein**
a protein is activated which contains an N-terminal helper sequence having one of the sequences specified in the following:

Met-Glu (SEQ ID NO:1)
Met-Thr-Pro-Leu-Pro-Arg-Pro-Pro (SEQ ID NO: 2)
Met-Thr-Pro-Leu-His-His-Pro-Arg-Pro-Pro
(SEQ ID NO: 3)
Met-Thr-Pro-Leu-Lys-Lys-Pro-Arg-Pro-Pro
(SEQ ID NO: 4)
Met-Thr-Pro-Leu-Glu-Glu-Gly-Pro-Arg-Pro-Pro
(SEQ ID NO: 5)
Met-Thr-Pro-Leu-Glu-Glu-Gly-Thr-Pro-Leu-Pro-Arg-
Pro-Pro (SEQ ID NO: 6)
Met-Thr-Pro-Leu-Glu-Glu-Gln-Pro-Pro (SEQ ID NO: 7)
Met-Lys-Ala-Lys-Arg-Phe-Lys-Lys-His-Pro-Arg-Pro-Pro
(SEQ ID NO: 8)
Met-Thr-Pro-Leu-Glu-Glu-Gly-Ile-Glu-Gly-Arg
(SEQ ID NO: 9)
Met-Thr-Pro-Leu-Lys-Ala-Lys-Arg-Phe-Lys-His-Pro-
Arg-Pro-Pro (SEQ ID NO: 10)

12. Process as claimed in one of the previous claims,
**wherein** a protein is activated said protein being granulocyte colony stimulating factor (G-CSF) or derivatives thereof.

## Revendications

1. Procédé pour activer des protéines recombinées qui sont présentes sous une forme au moins en partie inactive, caractérisé en ce que l'on active par des techniques de solubilisation et/ou de renaturation connues en soi une protéine qui contient à son extrémité N- et/ou C-terminale des séquences auxiliaires supplémentaires d'une longueur de 2 à 50 acides aminés, les hydrophobicités relatives de ces séquences auxiliaires, calculées comme étant la somme des hydrophobicités relatives des différents acides aminés indiquées dans le tableau 1, présentant une valeur numérique négative.

2. Procédé selon la revendication 1, caractérisé en ce que les séquences auxiliaires présentent une valeur du rapport de l'hydrophobicité relative au nombre des acides aminés de -2,0 kcal/mol ou inférieure.

3. Procédé selon l'une des revendications précédentes, caractérisé en ce que les séquences auxiliaires sont ajoutées à l'extrémité N-terminale de la protéine à activer.

4. Procédé selon la revendication 1 ou 2, caractérisé en ce que les séquences auxiliaires sont ajoutées à l'extrémité C-terminale de la protéine à activer.

5. Procédé selon l'une des revendications précédentes, caractérisé en ce que les séquences auxiliaires contiennent au moins 2 acides aminés du groupe consistant en le glutamate, l'aspartate, la lysine, l'arginine et la proline.

6. Procédé selon la revendication 5, caractérisé en ce que les séquences auxiliaires contiennent au moins deux restes glutamate.

7. Procédé selon la revendication 5 ou 6, caractérisé en ce que les séquences auxiliaires contiennent deux acides aminés chargés de même signe qui se suivent directement, du groupe consistant en le glutamate, l'aspartate, la lysine et l'arginine.

8. Procédé selon la revendication 7, caractérisé en ce que les séquences auxiliaires contiennent deux restes glutamate qui se suivent directement.

9. Procédé selon l'une des revendications précédentes, caractérisé en ce que l'on active des protéines qui présentent un site de clivage au niveau de la transition entre les séquences auxiliaires et la protéine voulue.

10. Procédé selon la revendication 9, caractérisé en ce que le site de clivage est un site de clivage d'IgA-protéase.

11. Procédé selon l'une des revendications précédentes, caractérisé en ce que l'on active une protéine qui contient une séquence auxiliaire N-terminale ayant une séquence indiquée dans la suite:

Met-Glu (SEQ ID NO: 1)
Met-Thr-Pro-Leu-Pro-Arg-Pro-Pro (SEQ ID NO: 2)
Met-Thr-Pro-Leu-His-His-Pro-Arg-Pro-Pro (SEQ ID NO: 3)
Met-Thr-Pro-Leu-Lys-Lys-Pro-Arg-Pro-Pro (SEQ ID NO: 4)
Met-Thr-Pro-Leu-Glu-Glu-Gly-Pro-Arg-Pro-Pro (SEQ ID NO: 5)
Met-Thr-Pro-Leu-Glu-Glu-Gly-Thr-Pro-Leu-Pro-Arg-Pro-Pro (SEQ
ID NO: 6)
Met-Thr-Pro-Leu-Glu-Glu-Gln-Pro-Pro (SEQ ID NO: 7)
Met-Lys-Ala-Lys-Arg-Phe-Lys-Lys-His-Pro-Arg-Pro-Pro (SEQ ID
NO: 8)
Met-Thr-Pro-Leu-Glu-Glu-Gly-Ile-Glu-Gly-Arg (SEQ ID NO: 9)
Met-Thr-Pro-Leu-Lys-Ala-Lys-Arg-Phe-Lys-Lys-His-Pro-Arg-Pro-
Pro (SEQ ID NO: 10)

12. Procédé selon l'une des revendications précédentes, caractérisé en ce qu'il s'agit, concernant la protéine que l'on active, du facteur stimulant les colonies de granulocytes (G-CSF) ou de dérivés de celui-ci.
